**Europäisches Patentamt**

**European Patent Office**

**Office européen des brevets**

(19)

(11) Numéro de publication : **0 351 296 B1**

(12)

# FASCICULE DE BREVET EUROPEEN

(45) Date de publication du fascicule du brevet :
**14.10.92 Bulletin 92/42**

(51) Int. Cl.⁵ : **A61K 47/00, A61K 9/16**

(21) Numéro de dépôt : **89401967.8**

(22) Date de dépôt : **10.07.89**

(54) **Procédé de préparation de microparticules de collagène et produits obtenus.**

(30) Priorité : **13.07.88 FR 8809544**

(43) Date de publication de la demande :
**17.01.90 Bulletin 90/03**

(45) Mention de la délivrance du brevet :
**14.10.92 Bulletin 92/42**

(84) Etats contractants désignés :
**AT BE CH DE ES FR GB GR IT LI LU NL SE**

(56) Documents cités :
**EP-A- 0 242 305**
**FR-A- 2 601 371**
**US-A- 4 671 954**
**IN VITRO CELLULAR & DEVELOPMENT BIO-**
**LOGY, vol. 21, no. 7, juillet 1985, pages**
**391-401, T'issue Culture Association, Inc.;K.W.**
**WISSEMANN et al.: "Pure gelatin micro-**
**carriers: Synthesis and use in cell attachment**
**and growth of fibroblast and endothelialcells"**

(73) Titulaire : **IMEDEX**
**Zone Industrielle des Troques**
**F-69630 Chaponost (FR)**
Titulaire : **PASTEUR MERIEUX SERUMS ET**
**VACCINS, Société Anonyme :**
**58 Avenue Leclerc**
**F-69348 Lyon Cédex 07 (FR)**

(72) Inventeur : **Dumas, Henri**
**10 Avenue de Ménival**
**F-69005 Lyon (FR)**
Inventeur : **Tayot, Jean-Louis**
**1 Rue des Greffières**
**F-69890 La Tour de Salvagny (FR)**

(74) Mandataire : **Bernasconi, Jean et al**
**CABINET LEMOINE ET BERNASCONI 13,**
**Boulevard des Batignolles**
**F-75008 Paris (FR)**

**Description**

La présente invention a trait à un procédé de préparation de microparticules ou microbilles de collagène.

La technique la plus couramment utilisée pour fractionner des solutions biologiques en microparticules est la mise en émulsion des solutions biologiques par dispersion dans une phase hydrophobe.

On peut citer, par exemple, le brevet américain n° US-A-3.092.553 (FISHER et WILSON) qui décrit un procédé permettant de réaliser des microbilles de gélatine incluant un principe actif pharmaceutique. Ce procédé est un système de goutte à goutte où la solution à fractionner tombe sur des vapeurs d'un liquide non miscible dans l'eau, dont la température est maintenue inférieure à la température de congélation du sol.

Il s'agit là d'une technique délicate à mettre en oeuvre et nécessitant de grandes quantités de liquide réfrigérant (azote liquide).

MIYATA TERUO et al (brevet européen n° 0.119.076) propose une technique de réalisation de microsphères en collagène fibrillé. Dans un premier temps, on produit des microsphères par dispersion d'une solution aqueuse acide de collagène dans un solvant organique non miscible dans l'eau (toluène ou chloroforme) pour réaliser une émulsion, puis, dans un deuxième temps, on solidifie les microsphères par alcalinisation du bain émulsionnant, par exemple par un mélange ammoniaque-éthanol.

Cette technique est coûteuse, car elle nécessite d'importantes quantités de réactifs.

Le brevet américain n° US-A-4.647.536 (MOSBACH et al) décrit la préparation de microporteurs sphériques en gélatine par émulsion chimique et que l'on réticule par le glutaraldéhyde. WISSEMAN et al dans In Vitro Cellular and Developmental Biology, Vol. 21, n° 7, p. 391 à 401 (1985) fait également état d'une technique de préparation de microporteurs en gélatine par mise en émulsion chimique utilisant le glutaraldéhyde comme agent réticulant.

La réticulation par le glutaraldéhyde présente des risques de rémanence, et donc de ralargage, de l'agent réticulant au cours de la dégradation du collagène (in vitro ou in vivo).

Il est également possible de fractionner des solutions biologiques en microparticules par nébulisation ou atomisation. Cette technique consiste à disperser une solution ou une suspension en fines gouttelettes dans un courant d'air chaud. Une telle technique est dénaturante pour le collagène par suite de l'utilisation de températures élevées (150°C à 200°C).

Il a été récemment présenté par DULLINGER, TAKENAKA et al (réunion du Club Collagène japonais, Université de Tokyo, 1988) une technique de cryobroyage appliquée à des solutions collagéniques dans lesquelles est incorporé un principe actif pharmaceutique. Cette technique présente, toutefois, un certain nombre d'inconvénients : obtention d'une poudre de granulométrie hétérogène, forme sphérique des particules non préservée, consommation importante de liquide réfrigérant.

La présente invention vise à remédier aux inconvénients précités, en proposant un procédé dans lequel on produit un jet de gouttelettes de collagène que l'on polymérise dans une solution aqueuse, par simple changement de pH.

L'un des buts de l'invention est de fournir des microbilles constituées d' un collagène non dénaturé, reticulées de façon homogène, qui soient d'une grande stabilité, biodégradables et sans toxicité chimique.

Un autre but de l'invention est de fournir des microbilles de collagène présentant, en surface, des groupements réactifs permettant le couplage du collagène avec des molécules biologiquement actives.

Un autre but encore de l'invention est de proposer un procédé de préparation de microbilles de collagène qui n'utilise ni solvants organiques, ni agents de réticulation autres que collagène, gélatine, polysaccharides naturels oxydés et où la polymérisation du collagène est obtenue en phase aqueuse par simple changement de pH.

Le procédé conforme à l'invention qui comporte la formation d'un jet de gouttelettes de collagène, par exemple par la technique dite de perlage, est caractérisé en ce que l'on forme les gouttelettes à partir d'une solution de collagène pouvant être préalablement soumise à un traitement en vue de faciliter la réticulation du collagène et conduisant à une solution fluide acide de collagène, en ce que l'on récupère les gouttelettes dans une solution aqueuse tamponnée à pH neutre ou basique, ladite solution induisant immédiatement la polymérisation du collagène , et en ce que l'on sépare les gouttelettes solidifiées de la solution et en ce que l'on lave lesdites gouttelettes.

Le collagène utilisé est du collagène humain ou bovin. On utilise avantageusement des solutions aqueuses de collagène humain de type I, III ou IV telles que celles qui ont été décrites dans les demandes de brevet n° FR-A-2 586 703 et n° FR-A-2 597 499 déposées respectivement les 2/9/1985 et 18/4/1986 au nom de l'INSTITUT MERIEUX ou encore des solutions aqueuses de collagène bovin de type I.

Ces solutions de collagène sont soumises à un traitement destiné à faciliter la réticulation du collagène, consistant à soumettre celles-ci à une oxydation ménagée.

En variante, on peut ne pas directement soumettre la solution de collagène à une oxydation ménagée, mais mélanger à celle-ci un polysaccharide tel que Dextran, cellulose, amidon préalablement soumis à une oxydation ménagée.

Pour effectuer l'oxydation ménagée, on aura avantageusement recours au procédé décrit dans la demande de brevet n° FR-A-2601371 déposée le 11/7/1986 au nom de l'INSTITUT MERIEUX. Selon le procédé décrit dans cette demande, le collagène est traité par une solution d'acide periodique ou de periodate notamment de sodium. Suivant une particularité de ce procédé, et dont il est fait utilisation dans la présente invention, on peut en jouant sur le pH, dans un premier temps, introduire une oxydation ménagée du collagène sans déclencher de réticulation (pH acide) puis, dans un second temps, à volonté par neutralisation ou alcalinisation du pH, déclencher la réticulation du collagène.

Selon le procédé conforme à la présente invention, on soumet la solution de collagène à une oxydation ménagée par l'acide periodique, cette oxydation s'effectuant par ajout à la solution de collagène d'une solution d'acide periodique jusqu'à l'obtention d'une concentration comprise entre $10^{-1}$ et $10\ M^{-4}$. On obtient, à l'issue de ce traitement, une solution fluide de collagène oxydé acide qui sert de solution de départ à la production des gouttelettes calibrées.

Les gouttelettes produites sont récupérées dans une solution aqueuse tamponnée à pH neutre ou basique qui induit immédiatement la réticulation du collagène.

On peut cependant, dans une autre forme de réalisation, éviter de procéder à un traitement préalable du collagène natif en récupérant les gouttelettes de collagène natif acide dans une solution neutre ou basique qui induit immédiatement une polymérisation superficielle permettant l'obtention d'une bille géométriquement stable et présentant des propriétés particulières.

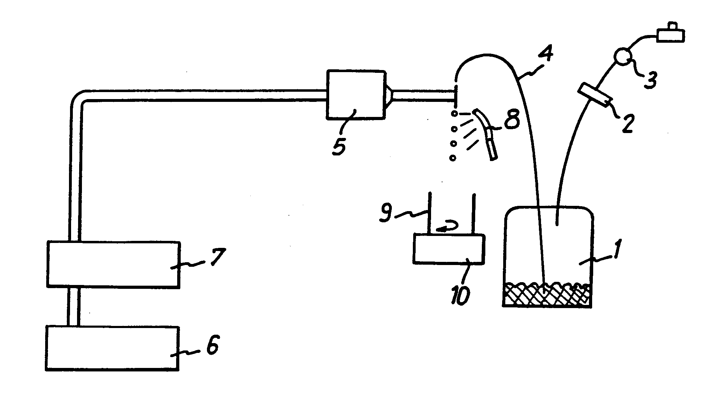

L'invention sera mieux comprise à la lecture du complément de description qui suit, se référant à la figure unique ci-annexée qui représente, de façon schématique, un dispositif de mise en oeuvre du procédé conforme à l'invention.

La solution de collagène qui a été, ou non, oxydée par traitement avec une solution d'acide periodique est placée dans le réservoir clos 1 dans lequel est envoyé de l'air comprimé que l'on filtre par l'élément filtrant 2 et dont on mesure la pression par le manomètre 3.

La solution de collagène circule sous pression dans le tuyau souple 4 qui est équipé à sa sortie d'un fin capillaire par lequel la solution de collagène oxydé s'écoule de façon laminaire.

Le capillaire est relié à un vibrateur 5 qui lui transmet un mouvement vibrant horizontal modulable. Le vibrateur 5 est lui-même associé à un générateur basse fréquence 6 et à un amplificateur 7.

A une fréquence déterminée, le jonc de collagène se déchire sous l'effet de la vibration. Il se forme alors dans l'air une succession de billes de diamètre constant.

Un stroboscope 8 permet de contrôler, en particulier, la sphéricité de ces billes qui s'écoulent à un fort débit.

Ces billes sont récupérées dans une solution aqueuse tamponnée qui induit la polymérisation immédiate des gouttelettes de collagène. Cette solution tamponnée est contenue dans le récipient 9 et agitée par l'agitateur magnétique 10.

Le recours à des changements de température ou l'emploi de solvants organiques sont, de ce fait, totalement inutiles. La structure en triple hélice du collagène est donc parfaitement conservée par le procédé conforme à l'invention qui n'est pas dénaturant pour les protéines.

Les billes alors stables, sont ensuite récupérées sur toile filtrante ou par centrifugation et lavées à l'eau distillée afin d'éliminer les réactifs éventuellement présents et le tampon.

Les billes obtenues ont une forme régulière sensiblement sphérique dont le diamètre moyen est généralement compris entre 50µm et 2 mm.

Ces billes sont constituées en un collagène non dénaturé, réticulées de façon homogène, biodégradables et sans toxicité chimique.

Ces microbilles présentent, à leur surface, des groupements réactifs (groupements aldéhydiques en excès provenant de l'oxydation du collagène) qui peuvent être utilisés pour le couplage de substances biologiquement actives.

Le procédé conforme à l'invention présente vis-à-vis des procédés antérieurs de nombreux avantages :

– il permet la production de microbilles calibrées en grandes quantités,

– il permet une production continue et automatisée,

– sa mise en oeuvre nécessite peu de matériel et de réactifs,

– il n'utilise ni solvants organiques, ni agents chimiques de réticulation tel que glutaraldéhyde,

– il n'est pas dénaturant pour le collagène.

Les microbilles obtenues par le procédé conforme à l'invention peuvent recevoir de nombreuses applications. Parmi celles-ci, on peut citer, par exemple :

– leur utilisation comme microporteurs de principes actifs par couplage avec des groupements aldéhydiques en excès, et qui n'ont pas été impliqués dans la réaction de réticulation,

– la préparation de médicaments à effet retard par inclusion, dans une matrice de collagène, d'un principe actif dont le relargage peut être contrôlé ; application qui est d'un intérêt particulier dans le cas où le principe actif est thermolabile ou sensible aux solvants grâce au caractère non dénaturant du procédé,

– leur utilisation comme microsupports pour culture cellulaire in vitro pouvant contenir d'autres molécules tissulaires incluses pendant la polymérisation des microbilles (glycosaminoglycanes, fibronectine...),

– inclusion de cellules dans les microbilles pouvant se faire aussi à l'aide d'un capillaire double, concentrique.

De façon avantageuse, la substance formant le principe actif est dissoute, à une concentration adéquate, dans la solution initiale de collagène.

On aura avantageusement recours aux microbilles conformes à l'invention pour toute présentation fine fractionnée d'une substance biologique (poudre hémostatique, produits d'hygiène corporelle ou d'activité cosmétique etc.).

Il va être donné, ci-dessous, à titre illustratif, quelques exemples de mise en oeuvre du procédé conforme à l'invention.

EXEMPLE 1.

La solution de départ est une solution aqueuse acide de collagène bovin type I à structure hélicoïdale préservée sans télopeptides, obtenue par déréticulation partielle de collagène de peaux de jeunes veaux (TRILLAGENE commercialisée par SADUC).

150 ml de cette solution à 1 % en $H_2O$ distillée à 20°C est oxydée pendant 2 heures à l'abri de la lumière et à 20°C par l'acide orthoperiodique afin d'obtenir une concentration finale de $5.10^{-3}$ M.

Après mise en place de cette solution dans l'appareil équipé d'un capillaire de diamètre intérieur de 280 µm, les microbilles sont produites à raison de 5 ml/mn (soit 15.000 billes/mn pour des billes d'un diamètre de 600µm et d'un volume de $1,1.10^{-4}$cm3).

Pendant 30 mn, les billes sont récupérées dans un tampon en rotation, tampon ($Na_2CO_3$ +$NaHCO_3$) 0,05 M - pH 9,3.

Les paramètres de la manipulation sont les suivants :
– pression d'air comprimé filtré : 1 atm
– fréquence du vibrateur : 500 Hz.

Ces billes sont récupérées sur une toile filtrante et lavées à l'eau distillée. Le produit final est une suspension de billes transparentes, d'un diamètre d'environ 600µm, représentant un volume de 180ml.

EXEMPLE 2.

On reprend l'exemple 1. La suspension de billes obtenue est déshydratée à l'acétone par bains successifs, puis sous hotte à flux laminaire (stérile ou non).

Le produit final est une poudre granuleuse d'environ 1,2 g, fine (de granulométrie inférieure à 400 µm et très hydrophile).

EXEMPLE 3.

La solution de départ est une solution acide de collagène placentaire humain de type III + I à 1 %. Cette solution est oxydée à l'acide periodique et subit les mêmes étapes que celles décrites dans l'exemple 1 (ou 1 et 2)

EXEMPLE 4.

On utilise le collagène type IV oxydé comme agent de réticulation.

Ce collagène type IV oxydé à 1 % est incorporé à raison de 20 % dans des solutions aqueuses à 1 % de collagène type IV (ou III + I). Les étapes de l'exemple 1 (ou 1 et 2) sont alors suivies.

EXEMPLE 5.

On reprend l'exemple 4, en utilisant le Dextran oxydé à la place du collagène IV oxydé.

Le Dextran est oxydé selon le même principe d'oxydation que le collagène type IV : par exemple, une solution de Dextran 500 à 5 % en eau distillée est traitée à l'acide periodique à raison d'une concentration finale de $2.10^{-2}$ M pendant 2 heures à l'abri de la lumière.

Cette solution peut être stérilisée par passage au travers d'un microfiltre 0,2 µm, conservée à +4°C et être utilisée comme agent de réticulation.

D'autres polysaccharides peuvent être utilisés : cellulose, amidon.

EXEMPLE 6.

La solution de départ est une solution de collagène bovin type I (solution aqueuse de collagène natif à 0,5 % : PANCOGENE de SADUC, société française). Les étapes de l'exemple 1 (ou 1 et 2) sont suivies.

EXEMPLE 7.

La solution de départ est une solution acide de collagène placentaire humain III + I (solution de collagène natif à 1 % en $H_2O$ distillée à 20°C).

Cette solution est mise en place dans l'appareil équipé d'un capillaire de diamètre intérieur 400 µm. Les microbilles sont produites à raison de 9 ml/mn (soit environ : 17.000 billes/mn pour des billes de diamètre 1 mm).

Les paramètres de la manipulation sont les suivants :
– pression d'air comprimé : 1,1 atm.
– fréquence du vibrateur : 215 Hz.

Ces billes sont récupérées dans une solution d'ammoniaque ($NH_4$ OH 0,1N) à pH 10,5. Il se produit une gélification instantanée des billes de collagène natif.

La solution d'ammoniaque contenant les billes de collagène en suspension est ramenée à pH 7-7,5.

Ces billes sont récupérées sur toile filtrante et lavées à l'eau distillée.

Le produit final est une suspension de billes de collagène opalescentes, d'un diamètre d'environ 1 mm.

EXEMPLE 8.

Les billes produites selon l'exemple 7 sont déshydratées à l'acétone en suivant les étapes de l'exemple 2.

EXEMPLE 9.

La solution de départ est une solution acide de collagène bovin de type I.

On reprend les étapes des exemples 7 (ou 7 et 8).

EXEMPLE 10.

Les billes produites selon l'un des exemples 1 et 3 à 7 sont lyophilisées pour donner une poudre divisée douée d'une grande capacité d'absorption d'eau.

EXEMPLE 11.

La solution de départ de l'exemple 1 est oxydée par l'acide orthoperiodique comme dans l'exemple 1.

Cette solution est ensuite passée dans un dispositif de goutte à goutte pour former des gouttes sphériques ou billes. Les billes, d'un diamètre de 2 mm environ sont récupérées dans le même tampon en rotation que dans l'exemple 1.

EXEMPLE 12.

La solution de départ est une solution acide de collagène bovin de type I. On reprend les étapes de l'exemple 9 à la différence que les billes d'un diamètre de 2,5 mm, ne sont pas produites par vibration mais par un dispositif de goutte à goutte usuel.

EXEMPLE 13.

On reprend les étapes de l'exemple 12 à la différence que dans la solution acide de collagène bovin de type I de départ, on a introduit un principe actif cosmétique. Ce principe actif est par exemple la superoxyde dismutase à une concentration de 10 U.I./ml dans cette solution.

Ces billes produites contiennent la superoxyde dismutase.

EXEMPLE 14.

La solution de départ est une solution aqueuse acide de collagène bovin de type 1.

Après mise en place de cette solution dans l'appareil équipé d'un capillaire de diamètre intérieur de 400 $\mu$m, les microbilles sont produites à raison de 9 ml/mn pour des billes de diamètre environ 1.1 mm.

Les paramètres de la manipulation sont les suivants :
. pression d'air comprimé : 1 atm.,
. fréquence du vibrateur : 200 Hz.

Les billes sont récupérées dans un tampon carbonate et/ou phosphate pH 9,3, et lavées selon l'exemple 1.

Le produit final est une suspension de sphéroïdes légèrement opalescents, de diamètre 0,9 - 1,3 mm.

EXEMPLE 15.

On peut avantageusement former des cristaux de phosphate de calcium dans des billes de collagène au moment de leur formation. Ceci peut être effectué, par exemple, de la façon suivante :
On reprend l'exemple 14.

La suspension de billes obtenues est incorporée, poids à poids, dans une solution de chlorure de calcium ($CaCl_2$) 0,5 M.

Après un temps de contact d'environ 30 mn, ces billes sont récupérées sur toile filtrante.

Les billes imprégnées de chlorure de calcium sont déversées dans une solution d'hydrogénophosphate disodique ($Na_2HPO_4$), 0,5 M.

Dès l'incorporation de ces billes dans la solution d'hydrogénophosphate disodique, il y a formation de cristaux de phosphate de calcium dans celles-ci.

Les billes sont alors lavées par bains successifs d'eau déminéralisée.

Les billes restent blanches, un squelette minéral de phosphate de calcium étant emprisonné de façon homogène entre les molécules de collagène. Ce squelette permet aux billes de posséder une résistance mécanique accrue.

EXEMPLE 16.

On reprend les étapes de l'exemple 1, à la différence que la solution de départ est une solution acide de collagène placentaire humain de type I à 1,5 %.

EXEMPLE 17.

On peut provoquer une contraction uniforme des billes de collagène augmentant leur densité en collagène, par irradiation suivie d'une élévation de température. Ceci peut être effectué, par exemple, de la façon suivante :
Les billes de collagène de type III + I réticulées, obtenues selon l'exemple 3, sont stérilisées, en suspension aqueuse, par rayonnement gamma à une dose de 2,5 Mrad.

Après irradiation, cette suspension de billes est chauffée à 37°C pendant 72 h.

L'observation morphologique montre une contraction des billes sans déformation, d'un facteur d'environ 1,5, c'est-à-dire :

. diamètre moyen avant irradiation 600µm,

. diamètre après irradiation, et 72 h à 37°C : 400µm.

Par ce procédé préservant la sphéricité des billes, il est possible d'intégrer des agents actifs qui migrent spontanément dans la bille avant contraction, puis qui restent emprisonnés après contraction, pour permettre un effet de libération retardée.

## Revendications

1. Procédé de préparation de microbilles de collagène à partir de gouttelettes d'une solution fluide, acide de collagène, caractérisé en ce qu'on forme un jet de gouttelettes calibrées que l'on recueille dans une solution aqueuse tamponnée à pH neutre ou basique, ladite solution aqueuse induisant immédiatement la polymérisation du collagène, et en ce qu'on sépare les gouttelettes solidifiées de la solution aqueuse, puis on les lave.

2. Procédé selon la revendication 1, caractérisé en ce que la solution de collagène a été soumise à un traitement en vue de faciliter la réticulation.

3. Procédé selon la revendication 2, caractérisé en ce que le traitement en vue de faciliter la réticulation du collagène consiste à soumettre tout ou partie de la solution de collagène à une oxydation ménagée.

4. Procédé selon la revendication 2, caractérisé en ce que le traitement en vue de faciliter la réticulation du collagène consiste à mélanger à la solution de collagène des polysaccharides préalablement soumis à une oxydation ménagée.

5. Procédé selon la revendication 4, caractérisé en ce que les polysaccharides sont choisis dans le groupe constitué par le Dextran, la cellulose, l'amidon.

6. Procédé selon l'une des revendications 3 à 5, caractérisé en ce que l'oxydation ménagée est effectuée par traitement par une solution d'acide periodique.

7. Procédé selon l'une quelconque des revendications 1 à 6, caractérisé en ce qu'une substance constituant un principe actif est disposée dans la solution de collagène de départ.

8. Procédé selon l'une quelconque des revendications 1 à 7, caractérisé en ce que les gouttelettes sont produites par vibration.

9. Procédé selon l'une quelconque des revendications 1 à 7, caractérisé en ce que les gouttelettes sont produites par un dispositif de goutte à goutte.

10. Procédé selon l'une quelconque des revendications 1 à 9, caractérisé en ce que l'on forme, dans les billes, des cristaux de phosphate de calcium.

11. Procédé selon l'une quelconque des revendications 1 à 10, caractérisé en ce que l'on soumet les billes a une irradiation puis à un chauffage pour provoquer une contraction des billes.

12. Microbilles de collagène caractérisées en ce qu'elles sont obtenues par application du procédé selon l'une quelconque des revendications 1 à 11.

13. Application des microbilles de collagène selon la revendication 12, comme microporteurs de principes actifs, dans la préparation de médicaments à effet retard, comme microsupports pour culture cellulaire, Comme produits de base cosmétique, en particulier dans l'élaboration de produits de gommage, de nettoyage cutané, comme agent de cicatrisation, comme agent hémostatique.

## Patentansprüche

1. Verfahren zur Herstellung von Kollagen-Mikrokugeln aus Tröpfchen einer flüssigen, sauren Kollagenlösung, dadurch gekennzeichnet, daß man einen Strahl kalibrierter Tröpfchen bildet, diesen in einer wäßrigen gepufferten Lösung mit neutralem oder basischem pH sammelt, wobei diese Lösung sofort die Polymerisation des Kollagens induziert, die erstarrten Tropfchen von der wäßrigen Lösung abtrennt und anschließend wäscht.

2. Verfahren nach Anspruch 1, dadurch gekennzeichnet, daß die Kollagenlösung einer Behandlung zur Erleichterung der Vernetzung unterworfen wurde.

3. Verfahren nach Anspruch 2, dadurch gekennzeichnet, daß die Behandlung zur Erleichterung der Vernetzung des Kollagens darin besteht, die Kollagenlosung ganz oder zum Teil einer schonenden Oxidation zu unterwerfen.

4. Verfahren nach Anspruch 2, dadurch gekennzeichnet, daß die Behandlung zur Erleichterung der Vernetzung des Kollagens darin besteht, der Kollagenlösung Polysaccharide beizumischen, die zuvor einer schonenden Oxidation unterworfen wurden.

5. Verfahren nach Anspruch 4, dadurch gekennzeichnet, daß die Polysaccharide ausgewählt sind aus der Gruppe von Dextran, Zellulose und Stärke.

6. Verfahren nach irgendeinem der Ansprüche 3 bis 5, dadurch gekennzeichnet, daß die schonende Oxidation durch Behandlung mit einer Perjodsäure-Lösung erfolgt.

7. Verfahren nach irgendeinem der Ansprüche 1 bis 6, dadurch gekennzeichnet, daß der Ausgangslösung von Kollagen eine Substanz hinzugefügt wird, die einen aktiven Wirkstoff darstellt.

8. Verfahren nach irgendeinem der Ansprüche 1 bis 7, dadurch gekennzeichnet, daß die Tröpfchen durch Vibration erzeugt werden.

9. Verfahren nach irgendeinem der Ansprüche 1 bis 7, dadurch gekennzeichnet, daß die Tröpfchen mittels einer Vorrichtung tropfenweise erzeugt werden.

10. Verfahren nach irgendeinem der Ansprüche 1 bis 9, dadurch gekennzeichnet, daß in den Kugeln Kalziumphosphatkristalle gebildet werden.

11. Verfahren nach irgendeinem der Ansprüche 1 bis 10, dadurch gekennzeichnet, daß man die Kugeln einer Bestrahlung mit anschließender Temperaturerhöhung unterwirft, um eine Kontraktion der Kugeln zu bewirken.

12. Kollagen-Mikrokugeln, dadurch gekennzeichnet, daß sie durch Anwendung des Verfahrens nach irgendeinem der Ansprüche 1 bis 11 erhalten werden.

13. Verwendung der Kollagen-Mikrokugeln nach Anspruch 12 als Mikroträger für aktive Wirkstoffe bei der Herstellung von Medikamenten mit Retard-Wirkung, als Mikroträger für Zellkulturen, als kosmetische Grundstoffe, insbesondere bei der Herstellung von Produkten für Körperpeeling und Produkten zur Hautreinigung, als Mittel zur Wundschließung und als Blutstillungsmittel.

**Claims**

1. A process for preparing collagen micropearls starting from droplets of acid fluid collagen solution, characterized in that a spray of calibrated droplets is formed which are collected in an aqueous solution buffered at neutral or basic pH, said aqueous solution immediately triggering the polymerisation of collagen, and in that the solidified droplets are separated from the aqueous solution and are subsequently washed.

2. A process according to claim 1, characterized in that the collagen solution has been subjected to a treatment in order to facilitate curing.

3. A process according to claim 2, characterized in that the treatment for facilitating collagen curing includes subjecting the whole or part of the collagen solution to a moderate oxidation.

4. A process according to claim 2, characterized in that the treatment for facilitating the curing of collagen includes mixing with the collagen solution polysaccharides which have been previously subjected to moderate oxidation.

5. A process according to claim 4, characterized in that the polysaccharides are chosen among the group including Dextran, cellulose and starch.

6. A process according to any of claims 3-5, characterized in that the moderate oxidation is carried out by treatment with a periodic acid solution.

7. A process according to any of claims 1-6, characterized in that a substance which is an active principle is placed in the starting collagen solution.

8. A process according to any of claims 1-7, characterized in that the droplets are produced by vibrations.

9. A process according to any of claims 1-7, characterized in that the droplets are produced with a drip device.

10. A process according to any of claims 1-9, characterized in that calcium phosphate crystals are formed in the pearls.

11. A process according to any of claims 1-10, characterized in that the pearls are subjected to radiation then to heating so as to cause a contraction of the pearls.

12. Collagen micropearls characterized in that they are obtained by application of the process according to any of claims 1-11.

13. Application of collagen micropearls according to claim 12, as microcarriers for active principles, to the preparation of sustained release drugs, as microsupports for cell culture, as cosmetic base products, particularly in the preparation of gumming products, products for cleaning the skin, as a healing agent, as a hemostatic agent.